(19) Europäisches Patentamt
European Patent Office
Office européen des brevets

(11) **EP 3 034 616 B1**

(12) **EUROPÄISCHE PATENTSCHRIFT**

(45) Veröffentlichungstag und Bekanntmachung des Hinweises auf die Patenterteilung:
**18.07.2018   Patentblatt 2018/29**

(21) Anmeldenummer: **15195686.9**

(22) Anmeldetag: **20.11.2015**

(51) Int Cl.:
*B01J 20/28* (2006.01)     *B01J 20/32* (2006.01)
*B01D 63/08* (2006.01)     *C12Q 1/24* (2006.01)
*G01N 33/543* (2006.01)    *B01J 20/02* (2006.01)
*B01J 20/10* (2006.01)

(54) **VORRICHTUNG ZUR HERSTELLUNG UND/ODER AUFREINIGUNG NANOPARTIKEL-GEKOPPELTER BIOMATERIALIEN UND VERFAHREN UNTER VERWENDUNG DERSELBEN**

DEVICE FOR PRODUCING AND/OR CLEANING NANOPARTICLE COUPLED BIOMATERIALS AND METHOD USING THE SAME

DISPOSITIF DE FABRICATION ET/OU DE NETTOYAGE DE BIOMATERIAUX COUPLES A DES NANOPARTICULES ET SON PROCEDE D'UTILISATION

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(30) Priorität: **17.12.2014   DE 102014118889**

(43) Veröffentlichungstag der Anmeldung:
**22.06.2016   Patentblatt 2016/25**

(73) Patentinhaber:
• **Salehi, Mohammad**
  **46236 Bottrop (DE)**
• **Hamann-Steinmeier, Angela**
  **49078 Osnabrück (DE)**

(72) Erfinder:
• **Salehi, Mohammad**
  **46236 Bottrop (DE)**
• **Hamann-Steinmeier, Angela**
  **49078 Osnabrück (DE)**

(74) Vertreter: **Scholz, Volker**
  **Boehmert & Boehmert**
  **Anwaltspartnerschaft mbB**
  **Pettenkoferstrasse 22**
  **80336 München (DE)**

(56) Entgegenhaltungen:
EP-A1- 2 085 464        WO-A1-88/01605
WO-A1-2013/056185       WO-A2-2005/093045
US-B2- 8 690 995

• **CHFER-PERICS C ET AL: "Functionalized inorganic nanoparticles used as labels in solid-phase immunoassays", TRAC, TRENDS IN ANALYTICAL CHEMISTRY, Bd. 31, 9. November 2011 (2011-11-09), Seiten 144-156, XP028345813, ISSN: 0165-9936, DOI: 10.1016/J.TRAC.2011.07.011 [gefunden am 2011-11-09]**
• **M. SAHELI ET AL.: "An Alternative Way to Prepare Biocompatible Nanotags with Increased Reproducibility of Results", J NANOMATER MOL NANOTECHNOL, vol. 5, no. 2, 20 April 2016 (2016-04-20), pages 1-5,**
• **DATABASE WPI Week 201421 Thomson Scientific, London, GB; AN 2013-X04356 -& CN 203 229 543 U (DIASYS DIAGNOSTIC SYSTEMS SHANGHAI CO LT) 9 October 2013 (2013-10-09)**

**Beschreibung**

Die vorliegende Erfindung betrifft eine Vorrichtung zur Herstellung und/oder Aufreinigung Nanopartikel-gekoppelter Biomaterialien und ein Verfahren unter Verwendung derselben.

Bei der Kopplung von Nanopartikeln an Biomoleküle, wie zum Beispiel Antikörper, kann es zu einem Aktivitätsverlust der Biomoleküle kommen. Um dies zu vermeiden, sind neue schonende Methoden im Herstellungsprozess von Nanopartikeln-gekoppelten Biomolekülen/Biomaterialien notwendig.

In der Nanotechnologie sind Nanopartikel zwischen 1 - 100 nm wegen ihres großen Oberflächen/Volumen-Verhältnisses als ideales Werkzeug für industrielle und auch für medizinische Applikationen von Interesse.

Die US-amerikanische Anmeldung US8690995-B2 enthüllt ein Verfahren zum Gewinnen von Nanopartikeln durch Verwendung einer Kammer, die eine mit einem Filter verschlossene Öffnung und einen Einlass zum Einleiten von Gas, Partikeln und Flüssigkeit umfasst.

Zur Lokalisation der Proteine auf Geweben wurden bisher verschiedene Methoden, basierend auf der oberflächenverstärkten Raman-Streuung (SERS, engl. surface enhanced Raman scattering), entwickelt, siehe z.B. M. Salehi, Entwicklung und Einsatz der Immun-SERS-Mikroskopie zur Gewebe-basierten Tumordiagnostik, Dissertation, Universität Osnabrück, (2013). Allerdings zeigen die auf Nanotechnologie basierenden Verfahren eine schlechte Reproduzierbarkeit. Ein bekanntes Problem in dieser Hinsicht ist der Verlust der Bioaktivität von Biomolekülen nach der Kopplung auf Oberflächen der Nanopartikel.

Während der Biokonjugation der Proteine an Nanopartikel wird in der Regel ein Überschuss von Proteinen (z.B. Antikörper) eingesetzt. Dieser Überschuss von Biomolekülen soll später durch Waschgänge (mit Hilfe von Zentrifugationsschritten) beseitigt werden. Dieses Problem tritt bei magnetischen Nano- oder Mikropartikeln, die viel schwerer sind, nicht auf.

Das Gewicht eines Nanopartikels steht in direkter relativer Korrelation zu seinem Volumen ($V = \frac{4}{3} . \pi r^3$). Bei der Herstellung von monodispersen Nanopartikeln ist eine Abweichung von ca. 10% des Volumens zu erwarten. Die Größenabweichung bei der Herstellung von z.B. 50 nm (R= 50 nm und r= 25 nm) großen Gold-Nanopartikeln beträgt $\pm$ 2,5 nm. Diese kleine Abweichung hat jedoch eine große Auswirkung auf das Gewicht der hergestellten Nanopartikel. Bei dem erwähnten Beispiel werden die Nanopartikel mit dem Durchmesser von ca. 45 nm etwa 27% leichter und die Nanopartikel mit dem Durchmesser von ca. 55 nm etwa 33% schwerer als die mit dem angezielten Durchmesser von 50 nm.

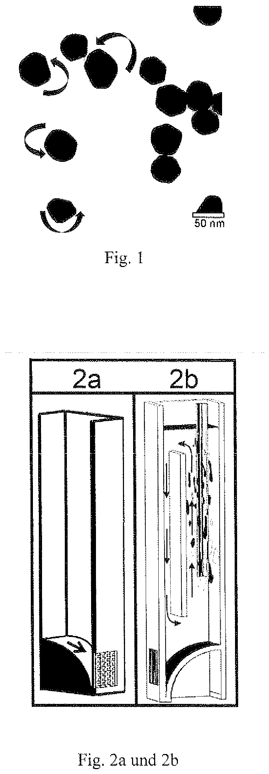

Die Nanopartikel werden während der Zentrifugation zwei verschiedene Rotationen übernehmen. Die erste Rotationsrichtung wird durch die Zentrifuge verursacht und die andere durch den eigenen Schwerpunkt der Nanopartikel. Dadurch drehen sich die Nanopartikel auch um die eigene Achse. Die Zentrifugalkraft beträgt in der Regel mehr als 2500 g. Das heißt, die Nanopartikel werden sich unter dem Einfluss der Zentrifugalkraft sowohl in verschiedene Richtungen drehen als auch gegenseitig überholen (wegen unterschiedlicher Form und Gewicht, S. Figur 1).

Es wird vermutet, dass die Zentrifugation der gemischten Proteine (Antikörper) und Nanopartikel mit so einer hohen Kraft zur Schädigung der gekoppelten Biomoleküle führt. Da ein Goldnanopartikel mit einem Durchmesser von 50 nm über 2000-fach schwerer als ein Antikörpermolekül ist, können gegenseitige Kollisionen unter dieser Zentrifugalkraft den empfindlichen Biomolekülen schaden. Große Biomoleküle, wie Antikörper, haben ohnehin die Neigung, Aggregate zu bilden. Abgesehen von physikalischen Schäden kosten die Zentrifugationsgänge viel Zeit. Während der Zentrifugation laufen die chemischen Reaktionen, die zur Bearbeitung der Nanopartikel notwendig sind, unkontrolliert weiter. Es ist daher die Aufgabe der vorliegenden Erfindung ein schonenderes Verfahren zur Trennung von Teilchen und/oder Molekülen unterschiedlicher Größe bereitzustellen, das Nachteile des Stands der Technik überwindet. Insbesondere soll eine Verfahren zur Trennung von Teilchen und/oder Molekülen unterschiedlicher Größe bereitgestellt werden, dass ohne Zentrifugationsschritte durchgeführt werden kann und eine Trennung erlaubt, die schneller und schonender durchgeführt werden kann, als mit Hilfe herkömmlicher Zentrifugationsverfahren.

Diese Aufgabe wird gelöst durch eine Vorrichtung wie beansprucht, umfassend eine Kammer mit zumindest einer Öffnung, wobei die Öffnung mit einem Filter verschlossen ist und wobei die Öffnung in einer der Seitenwände der Kammer derart angeordnet ist, dass zwischen der Öffnung und dem Boden der Kammer ein Endvolumen gebildet ist.

Erfindungsgemäß ist hierbei vorgesehen, dass zwischen der Öffnung und dem Boden ein gewisser Abstand besteht. Dieser Abstand führt dazu, dass in der Kammer unterhalb der Öffnung ein Raum gebildet wird. Dieser Raum kann als Endvolumen oder auch Totvolumen bezeichnet werden. Je nach Verwendungszweck kann die Größe des Endvolumens variiert werden. Soll das Endvolumen vergrößert werden, wird die Öffnung höher in der Seitenwand der Kammer angeordnet werden und umgekehrt.

Im erfindungsgemäßen Sinne soll eine Kammer als ein gefäßartiges Gebilde verstanden werden, das mit einer Flüssigkeit befüllt werden kann und das geeignet ist, diese Flüssigkeit aufzunehmen. Insbesondere umfasst die Kammer einen Boden und Seitenwände. In zumindest eine der Seitenwände ist die erfindungsgemäß vorgesehene Öffnung eingearbeitet. Das Endvolumen wird zwischen dem Boden der Kammer und der Öffnung gebildet. Insbesondere wird das Endvolumen zwischen dem unteren Ende der Öffnung und dem Boden der Kammer gebildet. Wird die Kammer vollständig mit einer Flüssigkeit befüllt, so fließt die Flüssigkeit, unter Einfluss der Schwerkraft, aus der Kammer durch die Öffnung heraus, bis der Flüssigkeitsspiegel das untere Ende der Öffnung erreicht hat. Die dann in der Kammer verbleibende Flüssigkeit füllt das Endvolumen der Kammer vollständig.

Durch die Verwendung der erfindungsgemäßen Vorrichtung kann eine schonende Trennung von Materialien unterschiedlicher Größe erreicht werden. Insbesondere wird durch die Verwendung der erfindungsgemäßen Vorrichtung zur Trennung der erwähnten Materialien eine schnelle (und dennoch schonende) Trennung gewährleistet.

Die Vorrichtung kann in einem Trennverfahren, das im Nachfolgenden im Detail beschrieben wird, verwendet werden. Die Trennung erfolgt hierbei insbesondere unter Einfluss der Schwerkraft. Abzutrennende Teilchen, die eine Abmessung haben, die größer als die Porengröße des Filters ist, werden nicht durch den Filter geleitet sondern durch Einwirkung der Schwerkraft auf diese Teilchen auf den Boden der Kammer absinken. Diese Teilchen sammeln sich folglich im Endvolumen. Angaben wie oberhalb, unterhalb, unter, seitlich und dergleichen beziehen sich folglich auf eine der Schwerkraft folgenden Anordnung. Der Boden der Kammer ist zum Erdboden hin ausgerichtet. Eine Öffnung oberhalb des Bodens ist weiter vom Erdboden entfernt als der Boden der Kammer.

Als Filter in der erfindungsgemäßen Vorrichtung ist ein gewöhnliches poröses Filtermaterial vorgesehen. Der Filter hat Poren. Die Größe der Poren (Porengröße) bestimmt hierbei, welche Materialien durch den Filter gelangen können und welche nicht. Es soll angenommen werden, dass ein Filter mit einer Porengröße von n Nanometern Materialien mit einem Durchmesser von bis zu n Nanometern passieren lässt, Materialien mit einem Durchmesser von mehr als n Nanometern hingegen zurückhält. Es ist vorgesehen, dass die erfindungsgemäße Vorrichtung einen ersten Einlass zum Einleiten einer Flüssigkeit in die Kammer und einen zweiten Einlass zum Einleiten eines Gases in die Kammer umfasst. Dadurch kann eine permanente Strömung in einer Flüssigkeit, die in der Kammer enthalten sein kann, erzeugt werden. Eine solche Strömung kann durch kontinuierlichen Zufluss einer Flüssigkeit, durch das Einblasen eines Gases, wie Stickstoff, $CO_2$ oder Luft, oder durch Kombination beider Maßnahmen erzeugt werden. Durch die Strömung entsteht eine Verwirbelung, mittels der ein Verstopfen des Filters vermindert oder vermieden werden kann.

Ebenso kann erfindungsgemäß bevorzugt vorgesehen sein, dass der Filter Poren einer Porengröße im Bereich von 10 bis 100 nm, vorzugsweise 10 bis 60 nm, besonders bevorzugt 10 bis 40 nm, insbesondere bevorzugt 1 bis 20 nm hat.

Durch geeignete Auswahl der Porengröße des Filters kann eine effiziente Trennung der zu trennenden Materialien ermöglicht werden.

Die Aufgabe wird ferner gelöst durch ein Verfahren zum Trennen einer ersten Gruppe von Teilchen und/oder Molekülen von einer zweiten Gruppe von Teilchen und/oder Molekülen, umfassend, a) Bereitstellen der ersten Gruppe von Teilchen und/oder Molekülen und der zweiten Gruppe von Teilchen und/oder Molekülen in der Kammer einer Vorrichtung nach einem der vorangehenden Ansprüche in Gegenwart einer Flüssigkeit, wobei die Teilchen und/oder Moleküle der ersten Gruppe und die Teilchen und/oder Moleküle der zweiten Gruppe in der Flüssigkeit zumindest teilweise gelöst und/oder dispergiert sind und die Teilchen und/oder Moleküle der ersten Gruppe eine Größe haben, die größer ist als eine Porengröße des Filters und die Teilchen und/oder Moleküle der zweiten Gruppe eine Größe haben, die kleiner ist als die Porengröße des Filters, und b) Durchleiten der Flüssigkeit durch den Fil-Der Begriff Teilchen soll in diesem Zusammenhang weit verstanden werden. Insbesondere soll der Begriff Teilchen Nano- und Mikropartikel, das heißt Partikel mit einem Durchmesser im Bereich von Nano- bzw. Mikrometern, umfassen. Ferner umfasst sein sollen insbesondere komplexe biologische Systeme, wie Zellen, Viren, Bakterien etc. Insbesondere soll verallgemeinert der Begriff Teilchen Nano- bzw. Mikromaterialien (Materialien mit einem Durchmesser im Bereich von Nano- oder Mikrometern) umfassen, die nicht als Molekül angesehen werden können, aber dennoch in erfindungsgemäßer Weise in einer Flüssigkeit gelöst oder dispergiert werden können.

In dem erfindungsgemäßen Verfahren ist vorgesehen, dass zumindest die kleineren Teilchen oder Moleküle (der zweiten Gruppe) in der Flüssigkeit zumindest teilweise gelöst oder dispergiert sind. Bevorzugt vorgesehen ist, dass die kleineren Teilchen oder Moleküle in der Flüssigkeit gelöst sind. Beim Durchleiten der Flüssigkeit durch den Filter werden die darin dispergierten oder gelösten Teilchen, die ein Größe haben, die kleiner ist als die Porengröße des Filters, durch den Filter hindurchgeleitet. Teilchen oder Moleküle, die eine Größe haben, die größer ist als die Porengröße des Filters werden hingegen in der Kammer zurückgehalten. Unter Einwirkung der Schwerkraft werden die größeren Teilchen oder Moleküle der ersten Gruppe auf den Boden der Kammer, das heißt in das Endvolumen der Vorrichtung, absinken. Dadurch, dass das Endvolumen unterhalb der Öffnung liegt, ist ausgeschlossen, dass sämtliche Flüssigkeit aus der Kammer entfernt wird. Die Teilchen und Moleküle der ersten Gruppe werden daher stets von Flüssigkeit (im Endvolumen) umgeben sein. Dies ist besonders dann vorteilhaft, wenn die erste Gruppe von Teilchen oder Molekülen Materialien, etwa Biomoleküle, sind, die lediglich in Kontakt mit einer Flüssigkeit stabil sind.

Teilchen sind erfindungsgemäß dann kleiner (bzw. größer) als die Porengröße des Filters, wenn die Teilchen in zumindest einer Raumrichtung, vorzugsweise in mehr als einer Raumrichtung, besonders bevorzugt in allen drei Raumrichtungen, einen Durchmesser aufweisen, der kleiner (bzw. größer) ist als der Durchmesser der Poren des Filters. Insbesondere gelten Teilchen dann als kleiner als die Porengröße des Filters, wenn ein Durchtritt der Teilchen durch die Poren des Filters möglich ist. Ebenso gelten die Teilchen als größer als die Porengröße des Filters, wenn diese nicht durch die Poren des Filters gelangen können.

Als Flüssigkeit können hierbei insbesondere Wasser oder wässrige Lösungen (anwendungsabhängig allerdings auch organische Lösungsmittel) vorgesehen sein. Bei der Trennung von Molekülen, die eine pH-Wert-abhängige Stabilität aufweisen (Biomoleküle, wie Proteine etc.), kann die Verwendung eines wässrigen Puffers-Systems als Flüssigkeit vorteilhaft sein.

Das erfindungsgemäße Verfahren ermöglicht eine schnelle und schonende Trennung von Teilchen und Molekülen der ersten Gruppe von Teilchen und Molekülen der zweiten Gruppe.

In einer bevorzugten Ausführungsform kann vorgesehen sein, dass das Durchleiten mittels eines Vakuums erfolgt, wobei ein Druckunterschied zwischen dem Inneren der Kammer und dem Äußeren der Kammer erzeugt wird und wobei der Druck im Inneren der Kammer größer ist als außerhalb der Kammer.

Die Unterstützung durch Durchleiten durch Anlagen eines Vakuums ermöglicht eine besonders schonende und schnelle Trennung der Teilchen und/oder Moleküle der ersten und der zweiten Gruppe. Ferner ergibt sich in dieser Ausführungsform der Vorteil, dass zu dem Zeitpunkt in dem der Flüssigkeitsspiegel in der Kammer die Höhe der Öffnung erreicht hat, das Vakuum unterbrochen wird. Auf diese Weise kann ein Ende der Trennung durch eine Begrenzung der Flüssigkeitsmenge in der Kammer oder eine Unterbrechung des Zuflusses an Flüssigkeit leicht vorgegeben werden.

In einer weiteren bevorzugten Ausführungsform kann vorgesehen sein, dass das Bereitstellen durch Binden zumindest eines Teils der Teilchen und/oder Moleküle der zweiten Gruppe an ein Vorläufermaterial erfolgt, wobei das resultierende Vorläufermaterial, an das Teilchen und/oder Moleküle der zweiten Gruppe gebunden sind, zumindest einen Teil der ersten Gruppe von Teilchen und/oder Molekülen ist.

In dieser bevorzugten Ausführungsform kann die Kammer der erfindungsgemäßen Vorrichtung als Reaktionskammer angesehen werden. Durch Reagieren des Vorläufermaterials mit den Teilchen oder Molekülen der zweiten Gruppe können Teilchen oder Moleküle der ersten Gruppe erhalten werden, die nach dem Vorgang des Anbindens in einfacher Weise von den in der Lösung oder der Dispersion verbleibenden Teilchen oder Molekülen der zweiten Gruppe abgetrennt werden können.

Diese Ausführungsform soll an einem konkreten Beispiel verdeutlicht werden. Erfindungsgemäß kann vorgesehen sein, dass es sich bei der Vorläuferverbindung um Nanopartikel handelt. Diese Nanopartikel sind in der Lage, an ihrer Oberfläche Moleküle der zweiten Gruppe zu binden. Beispielsweise kann es sich bei den Vorläuferverbindungen um Goldnanopartikel handeln. In diesem Fall kann vorgesehen sein, dass es sich bei den Molekülen der zweiten Gruppe um Thiole handelt, die in Kontakt mit Goldnanopartikeln (durch Bindung der SH-Funktionalität an das Gold) eine Monolage auf den Partikeln ausbilden. Das Vorläufermaterial, an das Teilchen und/oder Moleküle der zweiten Gruppe gebunden sind, sind in diesem Fall Goldnanopartikel, an die Thiole in Form einer Monolage gebunden sind.

In dieser bevorzugten Ausführungsform kann erreicht werden, dass gleich nach der Bindungsreaktion zwischen dem Vorläufermatrial und den Molekülen der zweiten Gruppen überschüssige Moleküle der zweiten Gruppe aus dem Reaktionsgemisch entfernt werden. Auf diese Weise ist eine einfache Steuerung der Reaktion möglich.

In einer weiteren bevorzugten Ausführungsform kann vorgesehen sein, dass die erste Gruppe von Teilchen und/oder Molekülen Nano- und/oder Mikropartikel, Bakterien, Viren, Plasmide oder Mischungen derselben umfasst.

Durch das erfindungsgemäße Verfahren ist es insbesondere möglich, instabile Nanopartikel und Biomolekule, wie die im Vorangehenden beschrieben, in schonender Weise zu trennen, ohne deren Struktur, etwa die Zellstruktur von Bakterien oder Viren, zu zerstören.

Ebenso kann erfindungsgemäß bevorzugt vorgesehen sein, dass die zweite Gruppe von Teilchen und/oder Molekülen niedermolekulare Verbindungen, Proteine, DNA, RNA, Lipide oder Mischungen derselben umfasst. Die im Vorangehenden aufgezählten Biomoleküle können durch das erfindungsgemäße Verfahren in einfacher und schonender Weise aus einem Gemisch mit größeren Materialien entfernt werden, ohne die Struktur der kleineren - häufig instabilen - Verbindungen zu zerstören.

Darüber hinaus wird die Aufgabe gelöst durch die Verwendung einer erfindungsgemäßen Vorrichtung zum Trennen einer ersten Gruppe von Teilchen und/oder Molekülen von einer zweiten Gruppe von Teilchen und/oder Molekülen, wobei die Teilchen und/oder Moleküle der ersten Gruppe eine Größe haben, die größer ist als eine Porengröße des Filters, und die Teilchen und/oder Moleküle der zweiten Gruppe eine Größe haben, die kleiner ist als die Porengröße des Filters.

Schließlich wird die Aufgabe gelöst durch die Verwendung einer erfindungsgemäßen Vorrichtung zum Herstellen von Nanopartikel-gebundenen Molekülen durch Reagieren von Nanopartikeln mit den Molekülen unter Ausbildung einer Bindung und anschließendem Abtrennen ungebundener Moleküle.

Überraschenderweise wurde durch die Erfinder herausgefunden, dass die im vorangehenden beschriebene einfache Anordnung eine schnelle, effiziente und dennoch schonende Trennung von Materialien unterschiedlicher Größe erlaubt. Bei der erfindungsgemäßen Lösung handelt es sich um eine Filtrationsmethode, die mithilfe von Vakuum funktioniert. Im Stand der Technik wird Zentrifugalkraft zur Filtration der Moleküle mit unterschiedlichen Größen eingesetzt. Solche Methoden können nicht zur Trennung von Nanopartikeln oder Zellen aus der Lösung dienen, da diese aufgrund der feinen Zellwände (besonders bei Mykoplasmen) während solcher Behandlungen geschädigt werden können.

Daher haben wir eine neue, schonende und schnellere Methode zur Trennung von Nanopartikeln aus einer Lösung entwickelt. Durch diese Methode wird auf Zentrifugationsgänge verzichtet und die Trennungszeiten werden drastisch reduziert.

Figur 2 zeigt den erfindungsgemäßen Weg, als Alternative für die Zentrifugation, zur Separation von nichtgebundenen Antikörpern (Große bevorzugt ca. 11 nm). Das System ist sehr robust und besteht aus einfachen Reaktionskammern mit seitlichen, vorzugsweise vertikalen, Filtermembranen, die Moleküle bis zu einer Größe von bevorzugt ca. 15 nm durchlassen. Am Ende jeder Säule befindet sich ein kleiner Behälter in Form einer Tasche, der als Totvolumen zur Ansammlung der Nanopartikel und des Puffers dient (Pfeil in Figur 2a).

Somit werden die mit Biomolekülen (z.B. Immunglobulin G) gebundenen Nanopartikel nie trocken. Der biologische Puffer wird mitsamt nicht gebundener IgG-Moleküle durch eine Vakuumpumpe abgesaugt. Dadurch werden die IgG-konjugierten Nanopartikel ohne Schaden von den nicht gebundenen IgG getrennt. Abgesehen davon haben Biomoleküle wie Enzyme eine zeitabhängige Lebensdauer. Die Zentrifugationsgänge nehmen viel Zeit in Anspruch, welche durch den Einsatz der neuen Filtrationsmethode deutlich verkürzt werden kann. Diese Zeit ist besonders bei der Bearbeitung von silicabeschichteten Nanopartikeln sehr wertvoll, da die optimalen Reaktionszeiten bei solchen Nanopartikeln sehr kurz sind (z.B. Aminofunktionalisierung beträgt 20 Minuten). Durch den hier neu vorgestellten Prozess werden diese Reaktionszeiten durch Zentrifugationen (weitere 20 Minuten) nicht überschritten.

Ein weiterer Vorteil der vorliegenden Erfindung ist die optionale oder bevorzugte permanente Strömung der Lösung im Reaktionsgefäß (s. Figur 2b). Diese Strömung wird durch einen kontinuierlichen Zufluss der Lösungen einerseits und durch ein Einblasen eines Gases (Stickstoff, CO2 oder Luft), erzeugt durch einen Automaten andererseits, gewährleistet. Durch die so entstehende Verwirbelung und die horizontalische Bewegung der Lösungen kann die Verstopfung des Filters durch metallische Nanopartikel vermindert werden.

Die Position des Filters ist in diesem System der einzige entscheidende Faktor für das Totvolumen bzw. das Endvolumen der gebildeten Seitentasche. Somit kann dieses Endvolumen beliebig rechnerisch vorbestimmt werden.

- Der Begriff "Reaktionsgefäß mit Seitenfilter" unterliegt erfindungsgemäß keinen besonderen Einschränkungen in Form oder Volumen des Reaktionsgefäßes und auch des gebildeten Totvolumens (Seitentasche).

- In diesem Verfahren wird bevorzugt Vakuum eingesetzt, um Proteine oder Nanopartikel vor Scherkräften am besten zu schonen. Das System kann jedoch erfindungsgemäß mit Überdruck oder einer Kombination aus Über- und Unterdruck (Vakuum) in Betrieb genommen werden.

- Die horizontale Bewegung der Lösungen jeglicher Art durch Druckdifferenz und die Bildung des mit filterpositionabhängigen Totvolumens für nicht-biologische Partikel (metallische, nicht metallische, Nano- oder Mikropartikel) und Biomoleküle (Proteine, DNA, RNA und Lipide) wird hier zum ersten Mal verwendet.

- Als Material für die Herstellung des Reaktionsgefäßes mit Seitenfilter können erfindungsgemäß und im Allgemeinen thermoplastische Kunststoffe eingesetzt werden. Beispiele hierfür sind Polyethylen, Polypropylen, Polyester, Polyamide, Polyvinylchlorid, Polycarbonat, Polymethacrylate, Polytetrafluorethylen, Polyimide, Polystyrol, Poly(meth)acrylate, Polyvinylidenfluorid, Fluorcopolymerisate, Elastomere wie beispielsweise natürliche oder synthetische Kautschuktypen oder Fluorelastomere.

- Die Reaktionsgefäße können für biomedizinische Applikationen beliebig mit Streptavidin, Protein A, Protein G, Protein AG, Protein L, Biotin oder Metallnanopartikeln beschichtet werden.

- Die Porengröße des Filters kann für die Nanotechnologie maßgeschneidert und für die Bearbeitung von metallischen Nanopartikeln kleiner als 100 nm ausgewählt werden. Die Größe der Poren unterliegt erfindungsgemäß keinen besonderen Einschränkungen und kann individuell verändert werden.

- Das Filtrationssystem kann erfindungsgemäß für die Bearbeitung einer Vielzahl von Biomaterialien wie, DNA, RNA, Lipide und Kohlenhydrate eingesetzt werden.

- Der Begriff "Nanopartikel" unterliegt erfindungsgemäß keiner besonderen Einschränkung in Form, Größe und Art der Materialien. In dieser Erfindung spricht man bevorzugt von metallischen Nanopartikeln in Größe von bevorzugt 1-100 nm. Diese können beschichtet oder auch unbeschichtet sein. Mit Beschichtungen sind Biomaterialien (wie DNA, RNA oder Proteine wie z.B. Antikörper, Protein A, Protein G, Protein L Streptavidin) oder Latex und Silica gemeint.

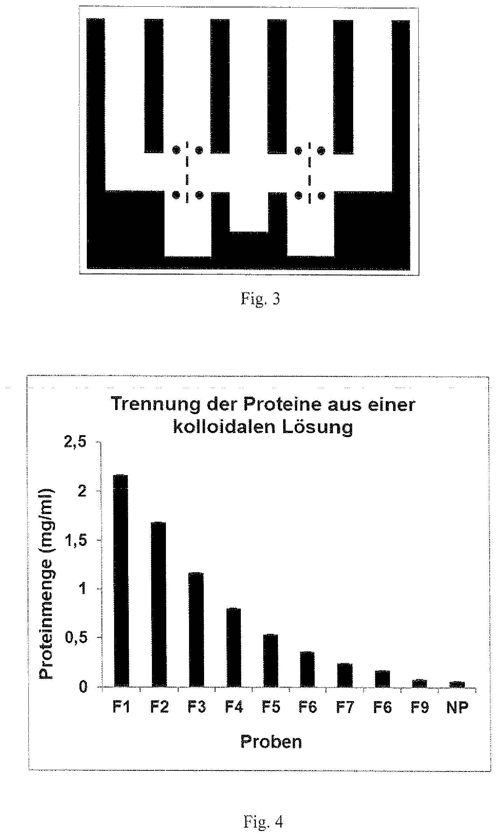

Das hier entwickelte Filtrations- bzw. Dialysesystem umfasst einzelne oder zwei verbundene Reaktionskammern und kann nach Bedarf auf drei oder mehr Kammern erweitert werden. Figur 3 zeigt ein Beispiel der vereinfachten Darstellung des Drei-Kammer-Systems. Im Prototyp wurden die Kammern mit Schrauben und Dichtungen zusammengefügt. Die Filter (als schwarze Striche dargestellt) sind zwischen den Dichtungen platziert (nicht dargestellt). Die Öffnungen auf den oberen Flächen der Kammern sind für die Überführung bzw. Entnahme der Proben und auch für den Anschluss der Vakuumpumpen vorgesehen.

Die Lösung zur Trennung der Nanopartikel wird in die erste Kammer (von links) überführt. Durch das Vakuum kann die Flüssigkeit durch ein Ventil von der mittleren oder letzten Kammer durch den Filter absaugt werden. Als Filter wurden verschiedene Membranen verwendet, die später näher beschrieben werden.

Im Folgenden soll Anhand von Ausführungsbeispielen die Erfindung unter Bezugnahme auf die Figuren weiter erläutert werden, wobei

Fig. 1. Monodisperse Nanopartikel mit Größenabweichungen. Die Drehrichtungen (Pfeil) sind nur als Beispiel dargestellt.

Fig. 2a: Die vereinfachte Darstellung des Filtrationssystems zur Entfernung von Biomolekülen durch das Vakuum. Durch das Vakuum sinkt die Lösung in der Kammer. Wenn die Lösung bis zur Grenze des Filters sinkt, wird das Vakuum unterbrochen. Somit läuft die Lösung nicht mehr aus der Kammer. Dadurch kann das Endvolumen der Lösung nach der Filtration vorher eingestellt werden.

Fig. 2 b: zeigt die schematische Darstellung der Verwirbelung von Lösungen im Filtrationssystem.

Fig. 3: schematische Darstellung des Filtrationssystem. Die Kammern werden durch Schrauben zusammengehalten. Zwischen den Kammern sorgen die Dichtungen für ein reibungsloses Vakuumieren.

Fig. 4: Die ermittelte Proteinmenge vor und nach der Dialyse. F1 stellt die Ausgangmenge in der ersten Kammer dar. Die Proben F2 bis F9 sind die ermittelten Proteinmengen des Durchlaufs in der zweiten Kammer. Das Vakuum wurde beim Prototypen nach dem Durchlauf von 1,5 ml Lösung in der zweiten Kammer ausgeschaltet um Messungen durchzuführen. Der NP-Balken zeigt die Menge von gebundenen BSA an Oberfläche von silicasverkapselten Nanopartikeln.

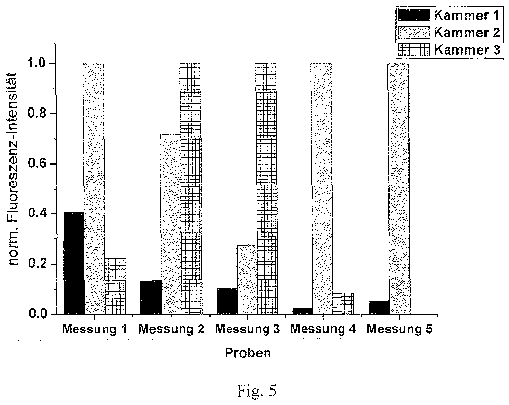

Fig. 5: normierte Fluoreszenzsignale der Proteinproben aus den drei verschiedenen Kammern.

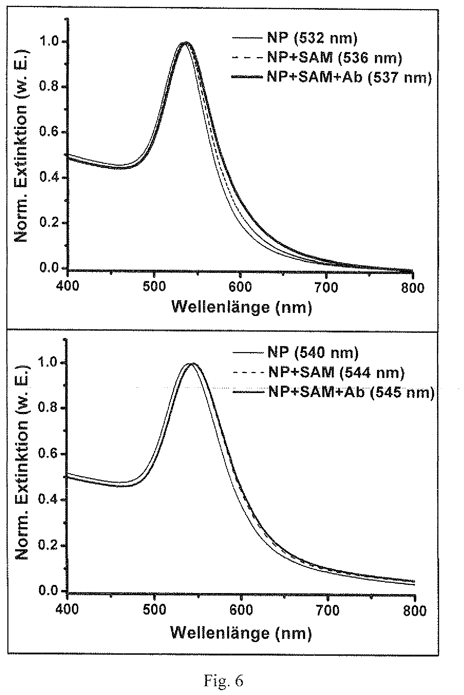

Fig. 6: Darstellung der Extinktionsspektren von Goldnanopartikeln (NP), Ramanmolekülbeschichteten Goldnanopartikeln (SAM) und IgG gekoppelten SAMs (NP+SAM+IgG). Extinktionsspektren der ca. 40 nm Goldkolloide vor und nach der Bindung an Antikörper in A und die von 60 nm Goldkolloide in B dargestellt. Die maximalen Extinktionswerte sind in Klammern angegeben.

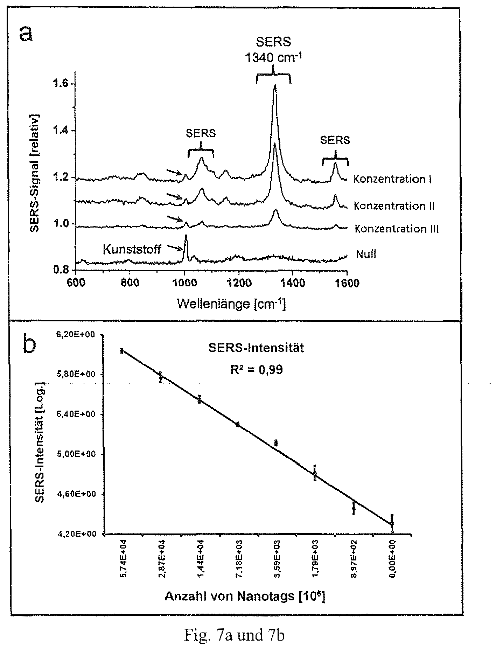

Fig. 7a: Darstellung der Ergebnisse von SRES-Messungen hinsichtlich der Bindungsaffinität von biokompatiblen Nanomaterialien mit immobilisierten Liganden auf Kunststoff. Die Bindungen zwischen Nanopartikeln und Kunststoff sind durch die Affinität von Streptavidin und biotinyliertem BSA zustande gekommen.

Ebenso kann erfindungsgemäß die Bindung durch die Affinität zwischen jeglichen Liganden und Rezeptoren erzielt werden. Dies beinhaltet insbesondere die Affinität der Antikörper und Antigen bei Bildung des Immunkomplexes, welcher bei ELISA-Verfahren der Fall ist.

Die internen Ramanbanden des Kunststoffs als Hintergrundsignale sind mit Pfeilen markiert. Durch internen Ramanbanden können sich die Intra- Interassay und Variationskoeffizienten der Verfahren verbessern,

Fig. 7b: Darstellung der Standardreihe des Versuches. Die Proben wurden 1:2 herunterverdünnt (Konzentration I-III). Die internen Ramanbanden (Kunststoff) sind mit Pfeilen markiert.

### Trennung von Nanopartikeln aus einer Proteinlösung

Im ersten Schritt wurde das Vakuumsystem zur Trennung von Rinderserumalbumin (BSA) aus einer kolloidalen Lösung eingesetzt.

Zuerst wurden nach Vorschriften von *Xia et. al* (Synthesis of Monodisperse QuasiSpherical Gold Nanoparticles in Water via Silver(I)-Assisted Citrate Reduction, Langmuir. 5, 3585-3589 (2010)) und *Perrault et. al* (Synthesis and Surface Modification of Highly Monodispersed, Spherical Gold Nanoparticles of 50-200 nm, J. Am. Chem. Soc. 47,17042-17043 (2009)) Goldnanopartikel mit einer Größe von 60 nm hergestellt. Danach wurde durch die Zugabe von einem Überschuss an Ramanreportermolekülen (4-Mercaptobenzoesäure) für die Ausbildung einer vollständigen selbstorganisierten Monolage (SAM) gesorgt. Die organischen Ramanmoleküle bilden eine stabile Gold-Schwefel-Bindung als Monoschicht auf der Oberfläche der Goldnanopartikel aus.

Die ramanmarkierten Nanopartikel wurden durch die Stöber-Methode mit Silica (Tetraethoxysilan) verkapselt. Vor dem Versuchsbeginn wurden die Nanopartikel mittels Crosslinker mit BSA beschichtet. Die Menge von gebundenem BSA auf den Nanopartikeln wurde mittels UV/VIS-Absorption/ Extinktionsspektroskopie bestimmt, wobei die nicht beschichteten Nanopartikel in dieser Messung als Blank (Referenz) dienten. Die Kammern wurden separat mit einer 0,3%igen Lösung von BSA in Boratpuffer über Nacht blockiert. Danach wurden die Nanopartikel im Boratpuffer mit einer BSA-Konzentration von 2,66 mg/ml aufgenommen. Alle Biofunktionalisierungsschritte der silicaverkapselten Nanopartikel wurden in einem neuen Dialysegefäß durchgeführt, hierbei wurde auf Zentrifugationen verzichtet.

3 ml von dieser Lösung wurden in die erste Dialysekammer überführt. Zur Trennung wurde ein Filter mit einer Porengröße von 40 nm eingesetzt. Nach Anschluss des Vakuums wurden UV/VIS-Werte des Durchlaufs in der zweiten Kammer (V= 1,5 ml) aufgenommen. Die ermittelten UV/VIS-Werte sind in Figur 4 dargestellt.

Die Proben wurden in Figur 4 als Fraktionen (F) bezeichnet. F1 ist die gesamte Proteinmenge als Ausganglösung in der Kammer 1. Darunter ist das nichtgebundene gelöste BSA und auch das an Nanopartikel gebundene zu verstehen. Die Menge von gebundenem BSA an Nanopartikel wurde vor der Überführung in die BSA-Lösung bestimmt und in den Figuren separat dargestellt (NP-Balken).

Durch den Einsatz des Prototyps konnte an drei unabhängigen Tagen eine 97,9%ige Aufreinigung des BSA erreicht werden.

Die Geschwindigkeit der Aufreinigung hängt von verschiedenen Faktoren ab, wie die Porengröße des Filters, Konzentration der Proteine in der Lösung (Viskosität), Temperatur der Lösung und des angelegten Unterdrucks.

Diese Geschwindigkeit des Lösungsdurchtritts durch die Membran wurde in dieser Studie bestimmt und als ml/Sekunden dokumentiert. Diese Geschwindigkeit hängt besonders von der Porengröße und Beschaffenheit des Filtermaterials ab. Im Schnitt konnte der Durchfluss der Lösung in unserem Prototyp mit einem Filterdurchmesser von ca. 8 mm bei einem Druck von 400 mbar zwischen 20 Sekunden (Poren für 150 kDa) bis 300 Sekunden (Poren für 25 kDa) ermittelt werden.

Diese Ergebnisse zeigen, dass die Methode auch zur schonenden Trennung von Zellen aus biologischen Flüssigkeiten wie, Fruchtwasser, Urin oder Zerebrospinalflüssigkeit verwendet werden kann.

Filtermembranen wurden von verschiedenen Firmen erworben. Der Versuch wurde mit folgenden Materialien ebenfalls erfolgreich durchgeführt.

Regenerated Cellulose (RC), Polyethersulfone (PES), Cellulose Nitrate (CN), Cellulose Acetate (CA), Polycarbonate Track-etched Membrane (PC-TEM), Polyamide (PA) und Polytetra-fluoroethylene (PTFE).

Im Folgenden soll der Anmeldungsgegenstand anhand von Ausführungsbeispielen unter Bezugnahme auf die Figuren weiter verdeutlicht werden, wobei
**Fig. 1.** Monodisperse
Nanopartikel mit Größenabweichungen. Die Drehrichtungen (Pfeil) sind nur als Beispiel dargestellt.

**Fig. 2a:** Die vereinfachte Darstellung des Filtrationssystems zur Entfernung von Biomolekülen durch das Vakuum. Durch das Vakuum sinkt die Lösung in der Kammer. Wenn die Lösung bis zur Grenze des Filters sinkt, wird das Vakuum unterbrochen. Somit läuft die Lösung nicht mehr aus der Kammer. Dadurch kann das Endvolumen der Lösung nach der Filtration vorher eingestellt werden.
**Fig. 2 b:** zeigt die schematische Darstellung der Verwirbelung von Lösungen im Filtrationssystem.

**Fig. 3:** schematische Darstellung des Filtrationssystem. Die Kammern werden durch Schrauben zusammengehalten. Zwischen den Kammern sorgen die Dichtungen für ein reibungsloses Vakuumieren.

**Fig. 4:** Die ermittelte Proteinmenge vor und nach der Dialyse. F1 stellt die Ausgangmenge in der ersten Kammer dar. Die Proben F2 bis F9 sind die ermittelten Proteinmengen des Durchlaufs in der zweiten Kammer. Das Vakuum wurde nach dem Durchlauf von 1,5 ml Lösung in der zweiten Kammer ausgeschaltet um Messungen durchzuführen. Der NP-Balken zeigt die Menge von gebundenen BSA an Oberfläche von silicasverkapselten Nanopartikeln.

**Fig. 5:** normierte Fluoreszenzsignale der Proteinproben aus den drei verschiedenen Kammern.

**Fig. 6:** Darstellung der Extinktionsspektren von Goldnanopartikeln (NP), Ramanmolekülbeschichteten Goldnanopartikeln (SAM) und IgG gekoppelten SAMs (NP+SAM+IgG). Extinktionsspektren der ca. 40 nm Goldkolloide vor und nach der Bindung an Antikörper in A und die von 60 nm Goldkolloide in B dargestellt. Die maximalen Extinktionswerte sind in Klammern angegeben.

**Fig. 7a:** Darstellung der Bindungsaffinität von biokompatiblen Nanomaterialien mit immobilisierten Liganden auf Kunststoff. Die Bindungen zwischen Nanopartikeln und Kunststoff sind durch die Affinität von Streptavidin und biotinyliertem BSA zustande gekommen. Ebenso kann erfindungsgemäß die Bindung durch die Affinität zwischen jeglichen Liganden und Rezeptoren erzielt werden. Dies beinhaltet insbesondere die Affinität der Antikörper und Antigen bei Bildung des Immunkomplexes, welcher bei ELISA-Verfahren der Fall ist. Die internen Ramanbanden des Kunststoffs als Hintergrundsignale sind mit Pfeilen markiert. Durch internen Ramanbanden können sich die Intra- Interassay und Variationskoeffizienten der Verfahren verbessern,

**Fig. 7b:** Stellt die Standardreihe des Versuches dar. Die Proben wurden 1:2 herunterverdünnt (Konzentration I-III). Die internen Ramanbanden (Kunststoff) sind mit Pfeilen markiert.

### Trennung der Proteine anhand der Größe

Die Reinheit des bindenden Proteins ist sehr wichtig für die Biofunktionalisierung der Nanopartikel, denn die Verunreinigung des Proteins mit Molekülen, wie BSA und Gelatine, die als Stabilisatoren eingesetzt werden oder mit Chemikalien wie Natriumazid (NaN3), können die Kopplung zwischen Nanopartikeln und Proteinen negativ beeinflussen. Solche Moleküle können primäre Amine beinhalten und dadurch selbst eine Bindung mit den Nanopartikeln eingehen.

Die von uns entwickelte Methode kann zur Entfernung der Stabilisatoren von Antikörpern eingesetzt werden. Da die Antikörper an sich zur Bildung von Aggregaten neigen, können durch diese Methode solche Aggregate gleichzeitig abgetrennt werden.

In Figur 5 ist das Resultat der Filtration von gemischten Antikörpern (IgG) und BSA dargestellt. In diesem Experiment wurde zuerst eine Mischung von Antikörpern und BSA in die erste Kammer überführt. Durch das Vakuum wurde die Flüssigkeit mit beiden Proteinen durch eine Filtermembran mit einer Porengröße von 150 kDa in die zweite (mittlere) Kammer gesaugt. Im nächsten Schritt wurde die Flüssigkeit mithilfe des Vakuums durch die zweite Filtermembran mit einer Porengröße von 100 kDa in die dritte Kammer gesaugt. Somit wurden die Antikörpermoleküle in der zweiten Kammer aufgefangen und weitere Moleküle bzw. BSA in die dritte Kammer überführt.

Das Vakuum wurde nach dem Durchlauf von ca. 1,5 ml Flüssigkeit in jeder Kammer ausgeschaltet, um die Menge der Proteine mit dem Fluoreszenzspektrometer ermitteln zu können. In der Ausgangslösung befanden sich 4 $\mu$g Antikörper gemischt mit 40 $\mu$g BSA. Am Ende konnte in der Kammer 1 ca. 0,3%, in der zweiten (mittleren) Kammer ca. 5,5% der gesamten Proteinmenge aufgefangen werden.

Die aufgefangenen Lösungen wurden einer SDS-PAGE unterzogen. Es konnten keine BSA Banden (65 kDa) in Kammer 1 und 2 gesichtet werden. Ebenso zeigte die Probe der Kammer 3 keine IgG-Banden (25 und 50 kDa), was bei der ersten und zweiten Kammer der Fall war.

Die ermittelten Fluoreszenzsignale wurden auf den maximalen Fluoreszenzwert der drei Messungen normiert, um den Verlauf des Durchflusses besser darstellen zu können.

### Schonende Kopplung der Antikörper auf Nanopartikel

Die isolierten IgG-Moleküle aus der zweiten Kammer wurden nach der fünften Messung auf der Oberfläche der Nanopartikel mit zwei verschiedenen Größen gekoppelt.

Dafür sollte die Oberfläche der Nanopartikel mit zwei verschiedenen Molekülen eine kovalente Bindung eingehen.

Es wurde eine ethanolische Lösung von 3 mM 5,5'-Dithiobis(2-nitrobenzoesäure) vorbereitet, die durch Zugabe von Natriumborhydridlösung (Ethanol) zu einer 4-Nitrothiobenzoe (4-NTB) säure reduziert wurde. Separat wurde auch ethanolische Lösungen von 0,3 mM Mercapto-undecansäure vorbereitet. Zur Ausbildung der Dual-Spacer-SAM wurden 180 $\mu$l 4-NTB mit 20 $\mu$l Mercapto-undecansäure gemischt. Mercapto-undecansäure dient als Bindestelle für Proteine nach Aktivierung mit N-Hydroxysulfosuccinimide Natriumsalz und 1-Ethyl-3-(3-dimethylamino-propyl) carbodiimide. Das detaillierte Vorgehen wurde in der vorherigen Arbeit beschrieben, M. Salehi, Entwicklung und Einsatz der Immun-SERS-Mikroskopie zur Gewebe-basierten Tumordiagnostik, Dissertation, Universität Osnabrück, (2013).

Die Proteinkopplung auf der Oberfläche der Nanopartikel mit Größen von ca. 40 und 60 nm wurde in einem neuen Reaktionsgefäß mit Seitenfilter durchgeführt, wobei auf Zentrifugations-schritte verzichtet wurde.

Das Ausmaß von Aggregation kann durch längerwellige Plasmonbanden (als zweiter Peak im Extinktionsspektrum) aufgrund der Plasmonkopplung bei Clusterbildung beobachtet werden. Bei einer optimalen Kopplung der Proteine an die Nanopartikel dürfte diese zusätzlichen langwelligeren Plasmonbanden im Extinktionsspektrum nicht zu sehen sein. Das Extinktionsmaximum sollte sich nicht mehr als um 8 nm verschieben.

Wie in Figur 6 ersichtlich ist, kann keine aggregationsbedingte Verschiebung von Plasmonbanden beobachtet werden. Die kleinen Verschiebungen der Plasmonbanden in Figur 6 kommen durch die Beschichtung der Ramanmoleküle oder Proteine zustande und nicht durch die Aggregationen.

### Proteinmessung anhand der Bindung der Liganden mit Rezeptoren und SERS-Signale

Die Proteinbestimmung kann direkt durch SERS-Signale der biokompatiblen Nanomaterialien ermittelt werden. Die Bindungen der Nanomaterialien an Zielmoleküle kommen durch Affinität der Liganden und Rezeptoren in jeglicher Art zustande.

Die zur Immobilisierung verwendete Oberflächen, zum Beispiel Kunststoffe, erzeugen selbst Raman-Signale, die permanent als Hintergrundsignale die Messungen begleiten. In Abb. 7A ist eines Hintergrundsignal bei 1000 cm$^{-1}$ mit einem Pfeil markiert. Die SERS-Spektren (bei ca. 1340 cm$^{-1}$) wurden auf die Kunststoffbande normiert.

Die Signalintensität des Kunststoffs ist bei drei Verdünnungen (Konzentration 1 bis Konzentration III) konstant, während die SERS-Signale sich ändern. Die Existenz solcher internen Kontrollen darf als eine zusätzliche Standardreihe verwendet werden. Bei Änderung der Belichtungszeit mit dem Laser, Temperatur oder Trübung der Probe ändert sich das Signal der internen Kontrolle und die Messwerte werden automatisch korrigiert.

Dadurch können sich die intra- Interassay und Variationskoeffizienten der Verfahren drastisch verbessern, was als Grundvoraussetzung für eine Standardisierung eines Verfahrens angesehen werden darf. Abb. 7B zeigt die resultierte Standardreihe des Versuches.

Die internen Ramanbanden können als Aussaß zur Quantifizierung der Proteine, Medikamente und Drogen der biomedizinischen verwendet werden. Diese sind Erfindungsgemäß als alternative zu kompetitivem ELISA vorgesehen.

Dadurch kann auf die Durchführung von Standardreihen verzichtet werden.

Das Absaugen der Flüssigkeiten durch das Vakuum verkürzt die 5 mal 15 minütigen notwendigen Zentrifugationen für die Bearbeitung und das Waschen der Nanopartikel auf 5 mal weniger als 40 Sekunden beim Prototypen. Somit wurde also eine Zeitersparnis von ca. 95% erreicht.

Die Existenz der Proteine auf der Oberfläche der Nanopartikel wurde durch Western Blot nachgewiesen.

Somit ist nachgewiesen, dass die Aggregation der Nanopartikel verhindert wird und dabei gleichzeitig ausreichend Proteine auf die Oberfläche der Nanopartikel kovalent gebunden werden.

Das hier vorgestellte Filtrationssystem kann neben der Aufreinigung und Bearbeitung der Nanopartikel und Proteine auch zur Dialyse eingesetzt werden und Bakterien, Viren und Plasmide von Lösungen trennen. Proteine können dadurch schonender konzentriert werden (wegen der Seitentasche bzw. des Totvolumen).

Die Immuntherapie kann durch den Einsatz der Antikörper mittels dieser Filtration sicherer durchgeführt werden, da die Biomoleküle wie Antikörper während der Lagerung Aggregate bilden können. Zurzeit werden die zur Therapie notwendigen Antikörper nach der Herstellung von Aggregaten befreit. Die Aggregate können bei therapeutischen Applikationen zu Nebenwirkungen führen. Über das Wachstum und die Bildung der kolloidalen Aggregate sind gründliche Untersuchungen durchgeführt. Skrdla hat 2012 über die Ähnlichkeit und Korrelation der Aggregatenbildung von metallischen Nanopartikeln (Ostwald Reifung) mit Proteinen berichtet, PJ. Skrdla, Roles of Nucleation, Denucleation, Coarsening, and Aggregation Kinetics in Nanoparticle Preparations and Neurological Disease, Langmuir. 10, 4842-57 (2012). In diesem Fall wäre eine Aufreinigung der Proteine (z.B. IgG) von Aggregaten direkt nach der Herstellung nicht nur sinnlos, sondern gar kontraproduktiv, da die Gründe der Aggregationsbildung von IgG-Molekülen durch die Trennung der Aggregate nicht beseitigt werden. Die IgG-Monomere sind in der Lage, in wenigen Stunden erneut Aggregate zu bilden. Das heißt, dass sich die gereinigten IgG-Monomere (Produkte) zur Bildung der neuen Aggregate einsetzen. Daneben ist zu bemerken, dass die Filtrtion im herkömmlichen Format mit Membranen, die eine Porengröße von ca. 100 nm haben, durchgeführt wird. Diese Membranen sind fähig, nur sehr große Aggregate von IgG-Molekülen auszufiltern. Die kleineren (z.B. Pantamere oder Hexamere) können nicht, wie es in unserer Methode der Fall ist, mit dieser Porengröße entsorgt werden. Die von uns vorgelegte Erfindung bietet somit die Möglichkeit, Antikörper direkt vor der Verwendung (in Klinik oder Labor) von Aggregaten zu trennen.

Die in der vorstehenden Beschreibung, den Ansprüchen und Zeichnungen offenbarten Merkmale der Erfindung können sowohl einzeln als auch in beliebiger Kombination für die Verwirklichung der Erfindung in ihren unterschiedlichsten Ausführungsformen wesentlich sein.

**Patentansprüche**

1. Vorrichtung zur Herstellung von biokompatiblen Nanomaterialien, umfassend eine Kammer mit zumindest einer Öffnung, wobei die Öffnung mit einem Filter verschlossen ist und wobei die Öffnung in einer der Seitenwände der Kammer derart angeordnet ist, dass zwischen der Öffnung und dem Boden der Kammer ein Endvolumen gebildet ist, und ferner umfassend einen ersten Einlass zum Einleiten einer Flüssigkeit in die Kammer und einen zweiten Einlass zum Einleiten eines Gases in die Kammer.

2. Vorrichtung nach Anspruch 1, wobei der Filter Poren einer Porengröße im Bereich von 10 bis 100 nm, vorzugsweise 10 bis 60 nm, besonders bevorzugt 10 bis 40 nm, insbesondere bevorzugt 1 bis 20 nm hat.

3. Verfahren zum Trennen einer ersten Gruppe von Teilchen und/oder Molekülen von einer zweiten Gruppe von Teilchen und/oder Molekülen, umfassend,

   a) Bereitstellen der ersten Gruppe von Teilchen und/oder Molekülen und der zweiten Gruppe von Teilchen und/oder Molekülen in der Kammer einer Vorrichtung nach einem der vorangehenden Ansprüche in Gegenwart einer Flüssigkeit, wobei die Teilchen und/oder Molekülen der ersten Gruppe und die Teilchen und/oder Molekülen der zweite Gruppe in der Flüssigkeit zumindest teilweise gelöst und/oder dispergiert sind und die Teilchen und/oder Moleküle der ersten Gruppe eine Größe haben, die größer ist als eine Porengröße des Filters und die Teilchen und/oder Moleküle der zweiten Gruppe eine Größe haben, die kleiner ist als die Porengröße des Filters, und
   b) Durchleiten der Flüssigkeit durch den Filter.

4. Verfahren nach Anspruch 3, wobei das Durchleiten mittels eines Vakuums erfolgt, wobei ein Druckunterschied zwischen dem Inneren der Kammer und dem Äußeren der Kammer erzeugt wird und wobei der Druck im Inneren der Kammer größer ist als außerhalb der Kammer.

5. Verfahren nach Anspruch 3 oder 4, wobei das Bereitstellen durch Binden zumindest eines Teils der Teilchen und/oder Moleküle der zweiten Gruppe an ein Vorläufermaterial erfolgt, wobei das resultierende Vorläufermaterial, an das Teilchen und/oder Moleküle der zweiten Gruppe gebunden sind, zumindest einen Teil der ersten Gruppe von Teilchen und/oder Molekülen ist.

6. Verfahren nach einem der Ansprüche 3 bis 5, wobei die erste Gruppe von Teilchen und/oder Molekülen Nano- und/oder Mikropartikel, Bakterien, Viren, Plasmide oder Mischungen derselben umfasst.

7. Verfahren nach einem der Ansprüche 3 bis 6, wobei die zweite Gruppe von Teilchen und/oder Molekülen niedermolkulare Verbindungen, Proteine, DNA, RNA, Lipide oder Mischungen derselben umfasst.

8. Verwendung einer Vorrichtung nach einem der Ansprüche 1 oder 2 zum Trennen einer ersten Gruppe von Teilchen und/oder Molekülen von einer zweiten Gruppe von Teilchen und/oder Molekülen, wobei die Teilchen und/oder Moleküle der ersten Gruppe eine Größe haben, die größer ist als eine Porengröße des Filters, und die Teilchen und/oder Moleküle der zweiten Gruppe eine Größe haben, die kleiner ist als die Porengröße des Filters.

9. Verwendung einer Vorrichtung nach einem der Ansprüche 1 bis 3 zum Herstellen von Nanopartikelgebundenen Molekülen durch Reagieren von Nanopartikeln mit den Molekülen unter Ausbildung einer Bindung und anschließenden Abtrennen ungebundener Moleküle.

10. Verwendung eines biokompatiblen Nanomaterials erhältlich unter Verwendung einer Vorrichtung nach einem der Ansprüche 1 oder 2 zur Bildung eines Raman- oder SERS-aktiven Ligand-Rezeptor Komplexes.

11. Verwendung nach Anspruch 9 umfassend das Auswerten der Ergebnisse mittels der internen Ramanbanden der zur Immobilisierung verwendeten Oberflächen, wie Nano- und Mikrobeads oder Filtrationsvorrichtung.

**Claims**

1. Device for producing biocompatible nanomaterials, comprising a chamber having at least one opening, the opening being sealed with a filter, and the opening being arranged in one of the side walls of the chamber such that an end volume is formed between the opening and the floor of the chamber, and further comprising a first inlet for introducing a liquid into the chamber and a second inlet for introducing a gas into the chamber.

2. The device as claimed in claim 1, wherein the filter has pores with a pore size in the range from 10 to 100 nm, preferably 10 to 60 nm, particularly preferably 10 to 40 nm, especially preferably 1 to 20 nm.

3. A method of separating a first group of particles and/or molecules from a second group of particles and/or molecules, comprising,

a) providing the first group of particles and/or molecules and the second group of particles and/or molecules in the chamber of a device as claimed in any of the preceding claims in the presence of a liquid, wherein the particles and/or molecules of the first group and the particles and/or molecules of the second group are at least partially dissolved and/or dispersed in the liquid and the particles and/or molecules of the first group are of a size greater than a pore size of the filter and the particles and/or molecules of the second group are of a size smaller than the pore size of the filter, and
b) passing the liquid through the filter.

4. The method as claimed in claim 3, wherein the action of passing through is performed by means of a vacuum, a pressure difference being generated between the interior of the chamber and the exterior of the chamber and the pressure in the interior of the chamber being greater than that outside the chamber.

5. The method as claimed in either of claims 3 or 4, wherein the step of providing is effected by binding at least part of the particles and/or molecules of the second group to a precursor material, the resulting precursor material to which the particles and/or molecules of the second group are bound being at least part of the first group of particles and/or molecules.

6. The method as claimed in any of claims 3 to 5, wherein the first group of particles and/or molecules comprises nanoparticles and/or microparticles, bacteria, viruses, plasmids or mixtures thereof.

7. The method as claimed in any of claims 3 to 6, wherein the second group of particles and/or molecules comprises low-molecular-weight compounds, proteins, DNA, RNA, lipids or mixtures thereof.

8. Use of a device as claimed in either of claims 1 or 2 for separating a first group of particles and/or molecules from a second group of particles and/or molecules, wherein the particles and/or molecules of the first group are of a size greater than a pore size of the filter, and the particles and/or molecules of the second group are of a size smaller than the pore size of the filter.

**9.** Use of a device as claimed in any of claims 1 to 3 for producing nanoparticle-bound molecules by reacting nanoparticles with the molecules, forming a bond and then separating unbound molecules.

**10.** Use of a biocompatible nanomaterial obtainable by using a device as claimed in either of claims 1 or 2 to form a Raman or SERS-active ligand-receptor complex.

**11.** Use as claimed in claim 9 comprising evaluating the results by means of the internal Raman bands of the surfaces used for immobilisation, such as nanobeads and microbeads or a filtration apparatus.

## Revendications

**1.** Dispositif de fabrication de nanomatériaux biocompatibles, comprenant une chambre dotée d'au moins un orifice, l'orifice étant bouché par un filtre et l'orifice étant agencé dans l'une des parois latérales de façon à ce qu'un volume final soit formé entre l'orifice et le fond de la chambre, et comprenant en outre une première entrée destinée à introduire un liquide dans la chambre ainsi qu'une seconde entrée destinée à introduire un gaz dans la chambre.

**2.** Dispositif selon la revendication 1, dans lequel la taille des pores du filtre se trouve dans la plage de 10 à 100 nm, de préférence de 10 à 60 nm, de manière encore plus préférentielle de 10 à 40 nm, et tout spécialement de 1 à 20 nm.

**3.** Procédé de séparation d'un premier groupe de particules et/ou molécules d'un second groupe de particules et/ou molécules, comprenant les étapes consistant à

a) préparer le premier groupe de particules et/ou molécules et le second groupe de particules et/ou molécules dans la chambre d'un dispositif selon l'une des revendications précédentes en présence d'un liquide, les particules et/ou molécules du premier groupe et les particules et/ou molécules du second groupe étant au moins partiellement dissoutes et/ou dispersées dans le liquide et la taille des particules et/ou molécules du premier groupe étant supérieure à la taille des pores du filtre, et la taille des particules et/ou molécules du second groupe étant inférieure à la taille des pores du filtre, et à
b) guider le liquide dans le filtre.

**4.** Procédé selon la revendication 3 dans lequel le guidage a lieu grâce à un vide, une différence de pression étant générée entre l'intérieur de la chambre et l'extérieur de la chambre et la pression étant plus grande à l'intérieur de la chambre qu'à l'extérieur.

**5.** Procédé selon la revendication 3 ou 4, la préparation se faisant en fixant au moins une partie des particules et/ou molécules du second groupe à un matériau précurseur, le matériau précurseur en résultant auquel sont fixées des particules et/ou molécules du second groupe constituant au moins une partie du premier groupe de particules et/ou molécules.

**6.** Procédé selon l'une des revendications 3 à 5, le premier groupe de particules et/ou molécules comprenant des nanoparticules et/ou des microparticules, des bactéries, des virus, des plasmides ou des mélanges de ces composants.

**7.** Procédé selon l'une des revendications 3 à 6, le second groupe de particules et/ou molécules comprenant des composés de faible poids moléculaire, des protéines, de l'ADN, de l'ARN, des lipides ou des mélanges de ces composants.

**8.** Utilisation d'un dispositif selon l'une des revendications 1 ou 2 visant à séparer un premier groupe de particules et/ou molécules d'un second groupe de particules et/ou molécules, la taille des particules et/ou molécules du premier groupe étant supérieure à la taille des pores du filtre, et la taille des particules et/ou molécules du second groupe étant inférieure à la taille des pores du filtre.

**9.** Utilisation d'un dispositif selon l'une des revendications 1 à 3 visant à fabriquer des molécules fixées à des nanoparticules au moyen de la réaction de nanoparticules avec les molécules pour former une liaison et ensuite séparer les molécules non fixées.

**10.** Utilisation d'un nanomatériau biocompatible disponible en utilisant un dispositif selon l'une des revendications 1 ou 2 afin de former un complexe ligand-récepteur actif en Raman ou en SERS.

**11.** Utilisation selon la revendication 9 comprenant l'analyse des résultats au moyen des bandes Raman internes des surfaces utilisées pour l'immobilisation, comme des nanobilles et microbilles de plastique ou un dispositif de filtration.

Fig. 1

Fig. 2a und 2b

Fig. 3

Fig. 4

Fig. 5

Fig. 6

Fig. 7a und 7b

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

• US 8690995 B2

**In der Beschreibung aufgeführte Nicht-Patentliteratur**

• *Langmuir,* 2010, vol. 5, 3585-3589
• *J. Am. Chem. Soc.,* 2009, vol. 47, 17042-17043
• Entwicklung und Einsatz der Immun-SERS-Mikroskopie zur Gewebe-basierten Tumordiagnostik. **M. SALEHI.** Dissertation. Universität Osnabrück, 2013
• *Langmuir.,* 2012, vol. 10, 4842-57